# EUROPÄISCHE PATENTANMELDUNG

(11) **EP 1 508 346 A1**
(43) Veröffentlichungstag der Anmeldung: **23.02.2005**
(21) Anmeldenummer: 04018655.3
(22) Anmeldetag: 06.08.2004
(51) Int. Cl.: A61M 5/50

(54) **Spritze mit einer Wiederverwendungsperre**

(30) Priorität: 16.08.2003 DE 20312677 U
(71) Anmelder: Schwarzbich, Jörg, 33615 Bielefeld (DE)
(72) Erfinder: Schwarzbich, Jörg, 33615 Bielefeld (DE)
(74) Vertreter: Wiebusch, Manfred

(57) **Zusammenfassung**

Spritze mit einem Kolben (12) und einem Zylinder (11), der am vorderen Ende einen Konus (20) aufweist, und mit einer Wiederverwendungssperre, die ein am Kolben (12) ausgebildetes Auslöseelement (30) aufweist, dadurch gekennzeichnet, daß das Auslöseelement (30) so am vorderen Ende des Kolbens (12) ausgebildet ist, daß es beim Einschieben des Kolbens (12) in den Zylinder (11) mit dessen Konus (20) zusammenwirkt.

## Beschreibung

Die Erfindung betrifft eine Spritze mit einem Kolben und einem Zylinder, der am vorderen Ende einen Konus aufweist, und mit einer Wiederverwendungssperre, die ein am Kolben ausgebildetes Auslöseelement aufweist.

Der Begriff der Wiederverwendungssperre umfaßt im Sinne der Erfindung auch einen Mechanismus, bei dem die Wiederverwendung nicht durch ein aktives Sperren oder Blockieren verhindert wird, sondern dadurch, daß ein für das Funktionieren der Spritze wesentlicher Teil funktionsuntüchtig gemacht wird.

Die Wiederverwendung gebrauchter Spritzen kann zur Verbreitung ernster Krankheiten wie Aids oder Hepatitis führen. Aus der britischen Patentanmeldung GB 23 15 414 A ist eine Einwegspritze nach dem Oberbegriff des Anspruchs 1 mit einer Wiederverwendungssperre bekannt. Die Spritze weist im Zylinder ein ringförmiges Element auf, bei dem es sich entweder um eine Nut in der Innenwand des Zylinders oder um einen Wulst handelt. Die Kolbenstange der Spritze weist Widerhaken in Form von seitlichen Flügeln auf, die ein Hineinschieben des Kolbens in die Spritze erlauben, jedoch beim Herausziehen der Kolbenstange in die Nut eingreifen oder an den Wulst stoßen und so die Kolbenstange blockieren. Der Kolben einer einmal verwendeten Spritze läßt sich dadurch nicht mehr weit genug zurückziehen, um die Spritze ein weiteres Mal zu verwenden. Nachteilig ist jedoch, daß die Spritze im Inneren des Zylinders einen Hinterschnitt, nämlich entweder die Nut oder den Bereich zwischen dem Konus und dem Wulst, aufweisen muß und deshalb schwierig zu entformen ist.

Aufgabe der Erfindung ist es, eine Spritze der eingangs genannten Art zu schaffen, deren Zylinder einfacher aufgebaut und einfacher herstellbar ist.

Diese Aufgabe wird erfindungsgemäß dadurch gelöst, daß das Auslöseelement so am vorderen Ende des Kolbens ausgebildet ist, daß es beim Einschieben des Kolbens in den Zylinder mit dessen Konus zusammenwirkt.

Bei der erfindungsgemäßen Spritze wird das Auslöseelement für die Wiederverwendungssperre einfach dadurch betätigt, daß es in den ohnehin an der Spritze vorhandenen Konus, z. B. den sogenannten Luer-Konus, eintritt. An dem Zylinder der Spritze sind deshalb keine Hinterschnitte mehr erforderlich. Gegebenenfalls können am freien Ende oder im Inneren des Konus Gegenelemente ausgebildet sein, die mit dem Auslöselement zusammenwirken. Auch diese Gegenelemente lassen sich jedoch aufgrund ihrer Lage am Ende oder an der sich verjüngenden Innenfläche des Konus ohne Hinterschnitte fertigen.

Die so aufgebaute Spritze hat verschiedene Vorteile. Der Zylinder läßt sich einfach herstellen, da er im wesentlichen einen gleichförmigen Innenquerschnitt ohne Nuten oder Wülste aufweisen kann. Gegenüber einer herkömmlichen Einwegspritze ohne eine Wiederverwendungssperre ist allenfalls eine geringfügige Modifikation am Konus notwendig. Der Zylinder weist also einen sehr einfachen Aufbau auf. Vorteilhaft ist außerdem, daß der Zylinder eine gleichmäßige Wandstärke aufweisen kann. Ein großer Vorteil ist, daß das Auslöseelement den im Konus vorhandenen Raum nutzt, so daß die Länge des Zylinders gegenüber einer Einwegspritze ohne Wiederverwendungssperre kaum vergrößert werden muß. Ein weiterer Vorteil besteht darin, daß die glatten Innenwände des Zylinders während der Injektion eine ruckfreie Bewegung des Kolbens und u. U. auch den Verzicht auf besondere Kolbendichtungen erlauben.

Vorteilhafte Ausgestaltungen der Erfindung ergeben sich aus den Unteransprüchen.

In einer ersten Ausführungsform wird das Auslöseelement durch mindestens eine elastische Zunge am vorderen Ende des Kolbens gebildet, die einen Widerhaken aufweist, der beim Hintergreifen einer auf der Innenseite des Konus ausgebildeten Stufe den Kolben gegen Zurückziehen verriegelt. Die Spritze ist dadurch gegen Wiederverwendung gesperrt. Die Länge des Zylinders braucht hier gegenüber einer herkömmlichen Einwegspritze nicht vergrößert zu werden, da die Zunge den im Konus vorhandenen Raum nutzt.

Bei der Herstellung des Zylinders mittels Spritzgießen kann die Stufe problemlos vom vorderen Ende des Konus her entformt werden.

Die Spritze kann eine Sollreißstelle zwischen dem Kolben und einer Kolbenstange aufweisen. Wird nach dem Verriegeln des Kolbens die Kolbenstange mißbräuchlich zurückgezogen, so reißt sie hinter dem Kolben ab. Da der Kolben in dem Zylinder verbleibt, ist die Spritze weiterhin gegen Wiederverwendung gesperrt.

In einer zweiten Ausführungsform weist die Spritze als Alternative oder Ergänzung zu dem oben beschriebenen Auslösemechanismus eine ringförmige Kolbendichtung auf, die am Kolben von einer Halterung gehalten ist, welche beim Auflaufen des Auslöseelements auf den Konus gelöst wird. Bei gelöster Halterung wird die Kolbendichtung vom Kolben nicht mitbewegt, so daß bei einem Zurückziehen der Kolbenstange in der Spritze kein Unterdruck aufgebaut werden kann. Eine Wiederverwendung der Spritze wird dadurch unmöglich gemacht. Vorteilhaft ist außerdem, daß ein herkömmlicher Spritzenkörper verwendet werden kann, der keinerlei Modifikationen aufweisen muß.

Bevorzugt ist die Kolbendichtung elastisch vorgespannt und dehnt sich beim Lösen der Halterung axial aus. Die Kolbendichtung kann sich dabei so verformen, daß sie bei einem nachfolgenden Zurückziehen der Kolbenstange, bei dem sich die Halterung beispielsweise wieder in ihre frühere Stellung bewegt, nicht von der Halterung erfaßt wird.

Alternativ oder zusätzlich ist das nachgiebige Element mit einem Verriegelungselement in einer Stellung verriegelbar, in der die Halterung gelöst ist. Aufgrund des so verriegelten nachgiebigen Elements wird bei einem Herausziehen des Kolbens die Kolbendichtung von der weiterhin gelösten Halterung nicht erfaßt und wird somit nicht mit dem Kolben mitbewegt.

Bevorzugt wird die Halterung durch einen scheibenförmigen Teil des Kolbens und mindestens einen radialen Vorsprung an dem Auslöseelement gebildet, die die Kolbendichtung axial unter Vorspannung halten. Die Halterung kann dann einfach dadurch gelöst werden, daß der radiale Vorsprung beim Auflaufen des Auslöseelements auf den Konus so weit zur Mittelachse der Spritze hin abgelenkt wird, daß er die Kolbendichtung nicht mehr erfaßt und die Kolbendichtung sich axial entspannt. Sobald danach durch Zurückziehen der Kolbenstange der Kolben von der Kolbendichtung abgezogen wird, ist die Spritze undicht, so daß kein Unterdruck mehr in der Spritze aufgebaut werden kann. Ferner ergibt sich auf diese Weise ein besonders einfacher Aufbau des Kolbens.

Bevorzugt weist die Kolbendichtung einen Innenwulst auf, der härter ist als der äußere Teil der Dichtung. Der härtere Innenwulst kann beispielsweise von vergleichsweise kleinen radialen Vorsprüngen der Halterung gehalten werden, während sich die Kolbendichtung auf der Seite des Kolbens auf größerer Fläche abstützen kann. Durch eine geeignet angepaßte Härte des Innenwulstes kann die Kolbenposition, an der die Halterung den Innenwulst freigibt, gezielt festgelegt werden.

Ausführungsbeispiele der Erfindung werden im folgenden anhand der Zeichnung erläutert.

Es zeigen:
- Fig. 1: eine erste Ausführungsform einer Spritze, deren Kolben Zungen mit Widerhaken aufweist;
- Fig. 2: die Spritze nach Figur 1 mit verriegeltem Kolben;
- Fig. 3: eine zweite Ausführungsform einer Spritze mit einer Kolbendichtung in einer Halterung;
- Fig. 4: die Spritze aus Figur 3 mit gelöster Halterung;
- Fig. 5: die Spritze aus Figur 4 mit zurückgezogenem Kolben;
- Fig. 6: eine Variante der Spritze nach Figur 4, mit einem Verriegelungselement zum Verriegeln der Zungen aneinander; und
- Fig. 7: einen Querschnitt durch die aneinander verriegelten Zungen.

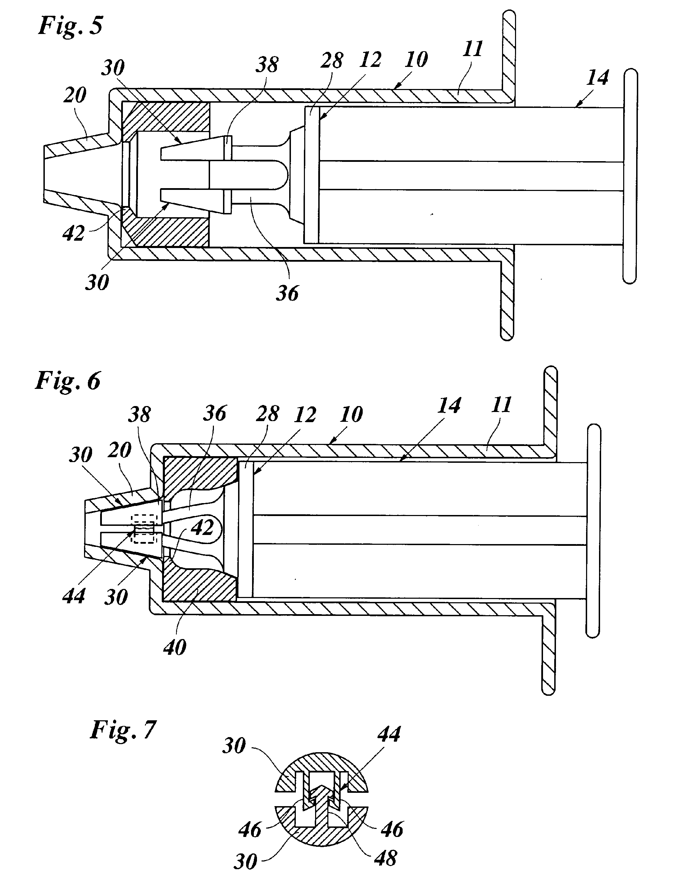

Mit Ausnahme von Figur 7 sind sämtliche Figuren Längsschnitte, bei denen allerdings der Kolben nicht aufgeschnitten ist.

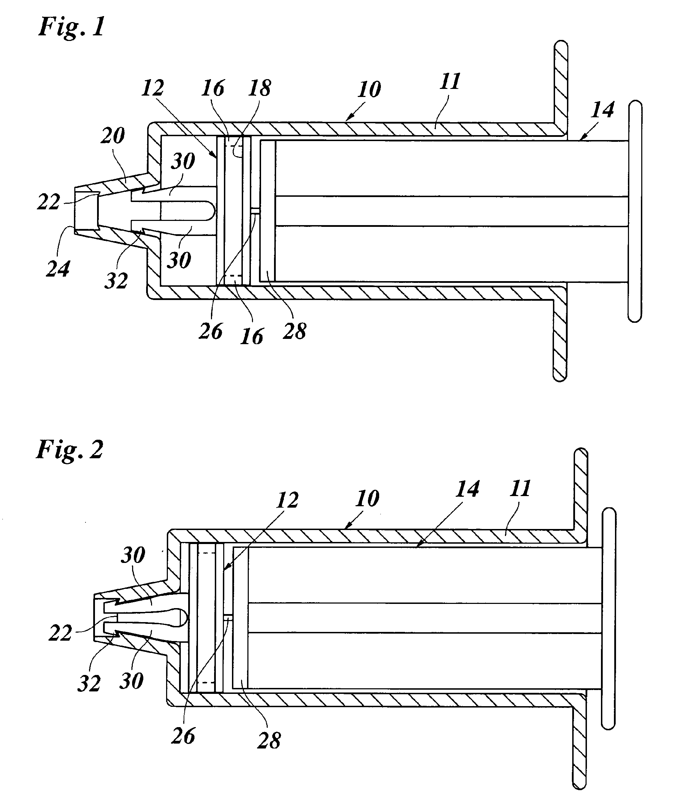

Die in Figuren 1 und 2 gezeigte Spritze besteht aus drei Teilen: einem Spritzenkörper 10, der einen Zylinder 11 bildet, einem Kolben 12, der eine Kolbenstange 14 aufweist, und einer Kolbendichtung 16, welche in einer ringförmigen Nut 18 des Kolbens 12 gehalten ist. Der Spritzenkörper 10 und der Kolben 12 mit der Kolbenstange 14 sind aus Kunststoff hergestellt. Die Kolbendichtung 16 kann aus Gummi hergestellt sein, sie kann aber auch aus Kunststoff oder in einem Stück mit dem Kolben 12 hergestellt sein.

Der Spritzenkörper 10 weist am vorderen Ende einen Kanülenansatz in Form eines Konus 20 auf. Nahe der Spitze des Konus 20 weist dieser eine ringförmige Stufe 22 auf, von der an der Konus 20 sich mit konstantem Innenradius bis zur vorderen Öffnung 24 des Konus 20 erstreckt.

Der Kolben 12 ist über einen Ansatz 26, der eine Sollreißstelle bildet, mit einer Scheibe 28 verbunden, die die Kolbenstange 14 vorne abschließt. Die Kolbenstange 14 hat einen kreuzförmigen Querschnitt. Ihr äußerer Radius ist kleiner oder gleich dem Innenradius des Zylinders 11.

Der Kolben 12 weist auf seiner Vorderseite zwei nachgiebige Auslöseelemente in Form von elastischen Zungen 30 auf, die an ihren vorderen Enden so geformt sind, daß sie in den Konus 20 eintreten können und dabei auf den Konus 20 auflaufen. An den Zungen 30 ist jeweils ein Widerhaken 32 ausgebildet, der beim weiteren Einschieben in den Konus 20 die komplementär geformte Stufe 22 hintergreift und so eine Wiederverwendungssperre auslöst.

Figur 2 zeigt die Situation nach dem Eingreifen der Widerhaken 32 hinter die Stufe 22, bei dem sich die elastischen Zungen 30 entspannen. Der Kolben 12 ist dadurch gegen Zurückziehen verriegelt. Wird dennoch unter größerer Kraftaufwendung an der Kolbenstange 14 gezogen, so reißt diese am Ansatz 26 vom Kolben 20 ab. In jedem Fall ist durch das Eingreifen der Widerhaken 32 hinter die Stufe 22 der Kolben 12 so gegen Zurückziehen verriegelt, daß er für eine erneute Verwendung der Spritze funktionsuntüchtig ist.

Eine denkbare Abwandlung dieser Ausführungsform besteht darin, daß in Umkehrung des oben beschriebenen Prinzips eine oder mehrere elastische Zungen an die Innenfläche des Konus angeformt sind und das Auslöseelement am Kolben Stufen bildet, an denen die Zungen verriegeln. Wegen der Verjüngung der Innenfläche des Konus sind auch in diesem Fall die Zungen ohne Hinterschnitte herstellbar.

Gemäß einer weiteren Abwandlung kann das Auslöseelement am Kolben im wesentlichen starr ausgebildet sein und mit einer Gegenkontur am Konus nach Art eines Druckknopfes zusammenwirken. In diesem Fall spürt der Benutzer einen Druckpunkt, unmittelbar bevor die Wiederverwendungssperre wirksam wird.

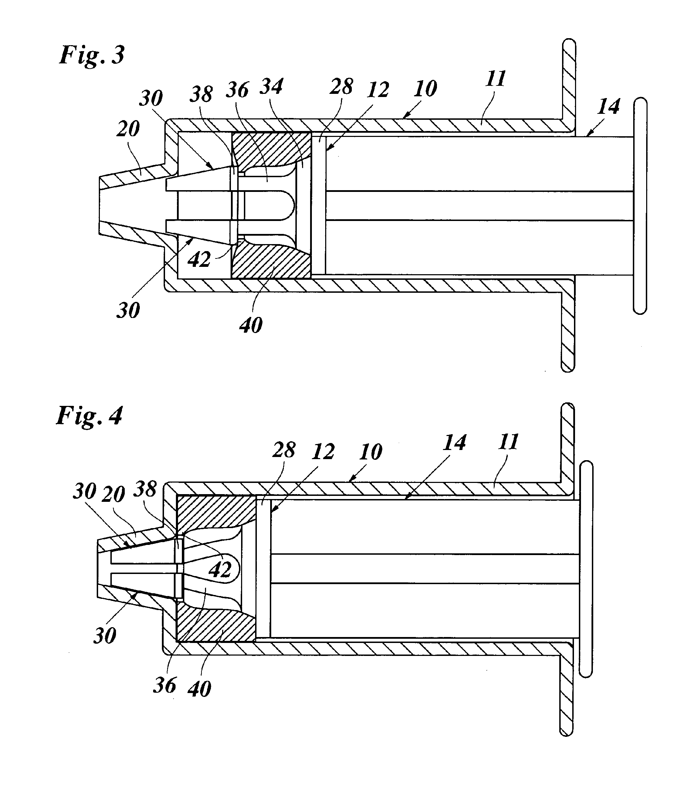

Die Figuren 3, 4 und 5 zeigen eine zweite Ausführungsform. Der Spritzenkörper 10 weist am Konus 20 keine Stufe auf, sondern entspricht einem herkömmlichen Spritzenkörper. An dem Kolben 12 sind an einem Sitz 34 auf der Scheibe 28 die Zungen 30 angeordnet. Die Zungen 30 weisen einen schlanken Teil 36 auf, an dessen vorderem Ende radiale Vorsprünge 38 ausgebildet sind. Von diesen Vorsprüngen aus verjüngen sich die Zungen 30 bis zu ihrer Spitze und sind dabei so geformt, daß sie sich in den Konus 20 bis etwa zum vorderen Ende des schlanken Teils 36 einführen lassen.

Die radialen Vorsprünge 38 bilden zusammen mit der Scheibe 28 eine Halterung für eine ringförmige Kolbendichtung 40, die sich mit ihrem einen Ende an der Scheibe 28 abstützt, sich an ihrem anderen Ende mit einem Innenwulst 42 an den radialen Vorsprüngen 38 der Zungen 30 abstützt und so die Scheibe 28 des Kolbens 12 gegen den Zylinder 11 des Spritzenkörpers 10 abdichtet. Die Kolbendichtung 40 ist aus einem elastischen Material gefertigt, beispielsweise aus Gummi oder einem geeigneten Polymer. Im Bereich des Innenwulstes 42 weist die Kolbendichtung 40 eine größere Härte auf als im Außenbereich. Dies stellt sicher, daß die in der Halterung axial vorgespannte Kolbendichtung 40 von den radialen Vorsprüngen 38 gehalten werden kann.

Beim Einschieben des Kolbens 12 mit der Kolbenstange 14 in den Spritzenkörper 10 laufen die Zungen 30 auf den Konus 20 auf und werden nach innen abgelenkt. In einer in Figur 4 gezeigten Position sind die Zungen 30 so weit nach innen abgelenkt, daß die radialen Vorsprünge 38 den Innenwulst 42 der Kolbendichtung 40 nicht mehr erfassen und die Halterung somit gelöst ist. Die elastische Kolbendichtung 40 entspannt sich in diesem Moment in axialer Richtung, so daß der Innenwulst 42 an der Stirnfläche des Spritzenkörpers 10 anliegt. Der Kolben 12 kann unter axialer Stauchung der Kolbendichtung 40 noch geringfügig weiter in den Spritzenkörper 10 eingeschoben werden. In jedem Fall werden beim Zurückziehen des Kolbens 12 die radialen Vorsprünge 38 der Zungen 30 den Innenwulst 42 der Kolbendichtung 40 nicht wieder erfassen. Die Kolbendichtung 40 wird daher im vorderen Teil des Spritzenkörpers 10 verbleiben, wie in Figur 5 zu sehen ist.

Der Außenradius der Scheibe 28 des Kolbens 12 ist geringer als der Innenradius des Zylinders 11 des Spritzenkörpers 10. Das Lösen der Halterung der Kolbendichtung 40 hat daher durch die Entspannung der elastischen Kolbendichtung 40 bewirkt, daß beim Herausziehen des Kolbens 12 die Kolbendichtung 40 den Kolben 12 nicht mehr gegen den Zylinder 11 des Spritzenkörpers 10 abdichtet. Der Kolben ist dadurch für eine erneute Verwendung der Spritze funktionsuntüchtig gemacht. In Figur 5 ist die Kolbendichtung 40 in ihrer axial vollständig entspannten Stellung zu sehen.

Figur 6 zeigt eine Variante der zweiten Ausführungsform, bei der die Halterung dadurch gelöst wurde, daß die hinreichend weit aneinander angenäherten Zungen 30 mittels einer Verriegelungsvorrichtung 44 in einer Stellung aneinander verriegelt wurden, in der die radialen Vorsprünge 38 beim Zurückziehen des Kolbens 12 frei durch den Innenwulst 42 der Kolbendichtung 40 hindurchtreten. Bei dieser Variante sind daher keine hohen Anforderungen an die Fertigungsgenauigkeit des Innenwulstes 42 zu stellen, da unabhängig von dem genauen Innenradius die Halterung jedenfalls dadurch gelöst wird, daß die Zungen 30 aneinander einrasten.

Die Verriegelungsvorrichtung 44, die in Figur 7 vergrößert im Querschnitt zu sehen ist, weist an einer Zunge 30 zwei elastische Verriegelungshaken 46 auf, die an einem Gegenstück 48 der anderen Zunge 30 einrasten.

Selbstverständlich kann die Verriegelungsvorrichtung 44 auch mit der anhand der Figuren 3 bis 5 beschriebenen Funktionsweise kombiniert werden. In diesem Falle ergibt sich durch die axiale Entspannung der Kolbendichtung 40 und das Verriegeln der Zungen 30 ein Mechanismus mit zwei unabhängig voneinander arbeitenden Systemen, die jeweils sicherstellen, daß die Halterung der Kolbendichtung 40 gelöst wird und dadurch der Kolben für eine erneute Verwendung der Spritze funktionsuntüchtig gemacht wird.

## Patentansprüche

1. Spritze mit einem Kolben (12) und einem Zylinder (11), der am vorderen Ende einen Konus (20) aufweist, und mit einer Wiederverwendungssperre, die ein am Kolben (12) ausgebildetes Auslöseelement (30) aufweist, **dadurch gekennzeichnet, daß** das Auslöseelement (30) so am vorderen Ende des Kolbens (12) ausgebildet ist, daß es beim Einschieben des Kolbens (12) in den Zylinder (11) mit dessen Konus (20) zusammenwirkt.

2. Spritze nach Anspruch 1, **dadurch gekennzeichnet, daß** das Auslöseelement durch mindestens eine elastische Zunge (30) gebildet wird, die einen Widerhaken (32) aufweist, der beim Hintergreifen einer Stufe (22) im Konus (20) den Kolben (12) gegen Zurückziehen verriegelt.

3. Spritze nach Anspruch 2, **gekennzeichnet durch** eine Sollreißstelle (26) zwischen dem Kolben (12) und einer Kolbenstange (14).

4. Spritze nach einem der Ansprüche 1 bis 3, **gekennzeichnet durch** eine Kolbendichtung (40), die am Kolben (12) von einer Halterung (38, 28) gehalten ist, welche beim Auflaufen des Auslöseelements (30) auf den Konus (20) gelöst wird.

5. Spritze nach Anspruch 4, **dadurch gekennzeichnet, daß** die Kolbendichtung (40) elastisch und derart vorgespannt ist, daß sie sich beim Lösen der Halterung (38, 28) axial ausdehnt.

6. Spritze nach Anspruch 4 oder 5, **dadurch gekennzeichnet, daß** das Auslöseelement (30) mit einem Verriegelungselement (44) in einer Stellung verriegelbar ist, in der die Halterung (38, 28) gelöst ist.

7. Spritze nach einem der Ansprüche 4 bis 6, **dadurch gekennzeichnet, daß** die Halterung durch ein Element (28) des Kolbens (12) und mindestens einen radialen Vorsprung (38) an dem Auslöseelement (30) gebildet wird und die Kolbendichtung (40) axial einspannt.

8. Spritze nach einem der Ansprüche 4 bis 7, **dadurch gekennzeichnet, daß** die Kolbendichtung (40) einen Innenwulst (42) aufweist, der härter ist als der äußere Teil der Kolbendichtung (40).
